# EUROPEAN PATENT APPLICATION

(11) **EP 3 381 906 A1**
(43) Date of publication of application: **03.10.2018**
(21) Application number: 17163007.2
(22) Date of filing: 27.03.2017
(51) Int. Cl.: C07D 235/18, C07D 417/04, C07D 263/57, C07D 277/44, C07D 277/66, C07D 209/48, A61P 35/00, A61K 31/4035, A61K 31/4184, A61K 31/428, C07C 309/29, C07C 309/35, C07C 317/14, C07C 233/65, C07C 57/46, C07C 63/04

(54) **COMPOUNDS FOR USE AS HEPARANASE INHIBITORS**

(71) Applicant: Leadiant Biosciences SA, 6850 Mendrisio (CH)
(72) Inventor: GIANNINI, Giuseppe, 00071 Pomezia (ROMA) (IT); MOR, Marco, 25016 Ghedi (BS) (IT); RIVARA, Silvia, 43015 Noceto (PR) (IT)
(74) Representative: Ghirardi, Valeria

(57) **Abstract**

The present invention relates to the use of compounds of formula [R - L₁ - Ar₁ - L - Ar]₂- X having an anti-heparanase activity, in particular for the treatment of diseases and disorders associated with a heparanase activity.

## Description

### FIELD OF THE INVENTION

The present invention relates to compounds having an anti-heparanase activity and to their use for the treatment of diseases and disorders associated with heparanase activity.

### BACKGROUND OF THE INVENTION

Heparan sulfate proteoglycans (HSPGs) are major components of the basement membrane and extracellular matrix. They are ubiquitous macromolecules associated with the cell surface and extracellular matrix (ECM) of a wide range of cells of vertebrate and invertebrate tissues (Bernfield et al. Annu. Rev. Biochem. 1999, 68, 729; lozzo R. V., San Antonio J. D. J. Clin. Invest. 2001, 108, 349; Kjellen L., Lindahl U. Annu. Rev. Biochem. 1991, 60, 443). HSPGs are a class of carbohydrate-modified proteins involved in key biological processes, including a role as co-receptors for a number of ligand molecules to regulate their signaling and distribution (Nakato H., Li J.P. Int. Rev. Cell. Mol. Biol. 2016, 325, 275).

Heparan sulfate (HS) is a component of cell surface and matrix-associated proteoglycans (HSPGs). A key function of HS is to bind and interact with signaling proteins, growth factors, plasma proteins, immune-modulators and other factors. In doing so, the HS chains and HSPGs are able to regulate protein distribution, bio-availability and action on target cells. Moreover, the HS chains ensure that a wide variety of bioactive molecules bind to the cell surface and ECM and thereby function in the control of normal and pathological processes, among which are morphogenesis, tissue repair, inflammation, vascularization and cancer metastasis (Billings P.C., Pacifici M. Connect Tissue Res. 2015, 56, 272). HS glycosaminoglycans bind to and assemble a multitude of ECM proteins (i.e., laminin, fibronectin, collagen type IV) and thereby contribute significantly to the ECM self assembly and integrity.

Heparanase is an endoglycosidase which cleaves heparan sulfate (HS) and hence participates in degradation and remodeling of the extracellular matrix (ECM), with a release of bioactive saccharide fragments and HS-bound growth factors and cytokines. Heparanase is preferentially expressed in human tumors and its over-expression in tumor cells confers an invasive phenotype in experimental animals. Heparanase upregulation correlates with increased tumor vascularity and poor postoperative survival of cancer patients. Heparanase is synthesized as a 65 kDa inactive precursor that undergoes proteolytic cleavage, yielding 8 kDa and 50 kDa protein subunits that heterodimerize to form an active enzyme.

Heparanase exhibits also non-enzymatic activities, independent of its involvement in ECM degradation.

There is growing evidence that heparanase upregulates expression of genes that participate in cancer metastasis and angiogenesis, glucose metabolism, immune response, inflammation and atherosclerosis, suggesting that heparanase belongs to an emerging class of proteins that play a significant role in regulating transcription in addition to their well-recognized extra-nuclear functions (Pisano C. et al. Biochem. Pharmacol. 2014, 89, 12; Ilan N. et al. Int. J. Biochem. Cell Biol. 2006, 38, 2018; Fux L. et al. Trends Biochem. Sci. 2009, 34, 511; Parish C.R. et al. Matrix Biol. 2013, 32, 228; Ramani V.C. et al. FEBS J. 2013, 280, 2294; Vlodavsky I. et al. Matrix Biol. 2013, 32, 241; Yang Y. et al. Cancer Res. 2010, 70, 8329).

In recent years, heparanase has attracted considerable attention as a promising target for innovative pharmacological applications. Indeed, heparanase appears to be involved in major human diseases, from tumors to chronic inflammation, diabetic nephropathy, bone osteolysis, thrombosis and atherosclerosis, in addition to more recent investigation in various rare diseases (Rivara S. et al. Future Med Chem. 2016, 8, 647).

In view of the above, compounds able to specifically modulate the activity of this enzyme are highly desired as a useful pharmacological option for many therapeutic indications. Since heparanase is involved in a very wide range of molecular pathways, modulation of the expression or activity of this enzyme is a viable therapeutic option.

Potent and selective heparanase inhibitors are therefore highly searched as therapeutic tools for those clinical indications in which heparanase inhibition is pharmacologically useful.

However, although in the last twenty years numerous efforts have been made in this sense and huge developments have been made in the knowledge of heparanase activity and functions, so far no drug able to inhibit or modulate heparanase functions has yet been registered (Rivara S. et al. Future Med Chem. 2016, 8, 647).

Some compounds are known in the art as heparanase inhibitors.

For example, WO2002060373 discloses indole derivatives and their use as heparanase inhibitors.

WO2004046123 discloses benzoxazole, benzthiazole and benzimidazole derivatives and their use as heparanase inhibitors.

WO02060867 discloses carbazole derivatives and their use as heparanase inhibitors.

In particular, three classes of heparanase inhibitors are known: active analogs of endogenous substances, synthetic small molecule compounds and natural products. In the literature, monoclonal antibodies and nucleic acid-based inhibitors are also mentioned as possible heparanase inhibitors.

Among these, only a few heparin derivatives have so far reached the stage of clinical trial (see some review: Ferro V. Mini Rev. Med. Chem. 2004, 4, 693; Jia L., Ma S. Eur. J. Med. Chem. 2016, 121, 209; Rivara S. et al. Future Med. Chem. 2016, 8, 647).

However, the heparin-derivatives macromolecules currently under clinical investigation have a high molecular weight.

Small molecules, instead, are particularly desirable due to their better pharmacokinetic properties. Furthermore, they can be administered orally thus resulting in an improved patient compliance.

Indeed, progress in the development of drugs able to inhibit heparanase activity has been limited also by the lack of efficient small molecule inhibitors.

Therefore, there is still the need of small molecules able to inhibit heparanase activity.

For example, in Pan et al. (Bioorganic Medicinal Chemistry Letters 2006 Jan 15;16(2):409-12, 1-[4-(1H-Benzoimidazol-2-yl)-phenyl]-3-[4-(1H-benzoimidazol-2-yl)-phenyl]-urea derivatives as small molecule heparanase inhibitors. Pan W, Miao HQ, Xu YJ, Navarro EC, Tonra JR, Corcoran E, Lahiji A, Kussie P, Kiselyov AS, Wong WC, Liu H.), a class of 1-[4-(1H-benzoimidazol-2-yl)-phenyl]-3-[4-(1H-benzoimidazol-2-yl)-phenyl]-ureas is described and heparanase inhibitory activity of some compounds of this class is investigated. In Xu et al. (Bioorganic Medicinal Chemistry Letters 2006 Jan 15;16(2):404-8, N-(4-{[4-(1H-Benzoimidazol-2-yl)-arylamino]-methyl}-phenyl)-benzamide derivatives as small molecule heparanase inhibitors. Xu YJ, Miao HQ, Pan W, Navarro EC, Tonra JR, Mitelman S, Camara MM, Deevi DS, Kiselyov AS, Kussie P, Wong WC, Liu H) a class of N-(4-{[4-(1H-benzoimidazol-2-yl)-arylamino]-methyl}-phenyl)-benzamides is described and some compounds are tested as heparanase inhibitors.

In "3D QSAR based design of novel substituted urea molecules as heparanase inhibitors" (Raju Bathini, Sabiha Fatima, Sree Kanth Sivan, Vijjulatha Manga; Journal of Pharmacy Research Volume 7, Issue 8, August 2013, Pages 754-761) symmetric urea derivatives are designed and their activity on heparanase inhibition is predicted.

In Courtney et al., 2005 (Bioorg Med Chem Lett. 2005 May 2;15(9):2295-9, Furanyl-1,3-thiazol-2-yl and benzoxazol-5-yl acetic acid derivatives: novel classes of heparanase inhibitor, Courtney SM, Hay PA, Buck RT, Colville CS, Phillips DJ, Scopes DI, Pollard FC, Page MJ, Bennett JM, Hircock ML, McKenzie EA, Bhaman M, Felix R, Stubberfield CR, Turner PR), a series of furanyl-1,3-thiazol-2-yl and benzoxazol-5-yl acetic acids are synthetized and studied for their anti-heparanase activity.

In Courtney et al., 2004 (2,3-Dihydro-1,3-dioxo-1H-isoindole-5-carboxylic acid derivatives: a novel class of small molecule heparanase inhibitors. Courtney SM, Hay PA, Buck RT, Colville CS, Porter DW, Scopes DI, Pollard FC, Page MJ, Bennett JM, Hircock ML, McKenzie EA, Stubberfield CR, Turner PR. Bioorg Med Chem Lett. 2004 Jun 21;14(12):3269-73) a class of 2,3-dihydro-1,3-dioxo-1H-isoindole-5-carboxylic acids is disclosed as heparanase inhibitors.

It has now been found that compounds already known in the art but for which an activity on heparanase was never investigated before are endowed with heparanase inhibitory activity. Therefore, they can be advantageously used for the treatment of diseases related to heparanase activity.

### SUMMARY OF THE INVENTION

It is an object of the present invention a compound having the following formula (I)

[R-L₁-Ar₁-L-Ar]₂-X (I)

wherein
X is selected from the group consisting of NHCONH, O, SO₂, C₁-C₄alkylene, linear or branched, and 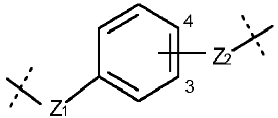 wherein Z₁ and Z₂ are the same or different and each of Z₁ and Z₂ is independently selected from the group consisting of O, S and CONH or it is absent, and Z₂ is in position 3 or 4 of the phenyl ring,
each of Ar and Ar₁ is independently selected from the group consisting of 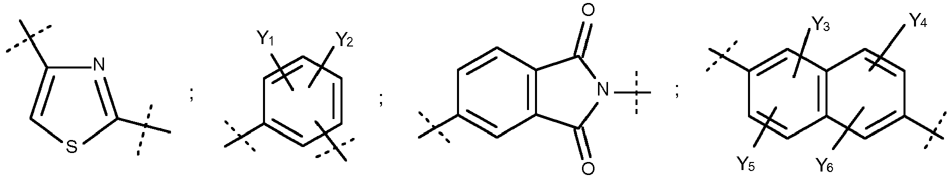
   or is absent, with the proviso that at least one of Ar and Ar₁ is present,
   each of L and L₁ is independently selected from the group consisting of N=N, NHCO, CONH, CO, S and O, or is absent,
R is selected from the group consisting of COOH, halogen, CH=CHCOOH, 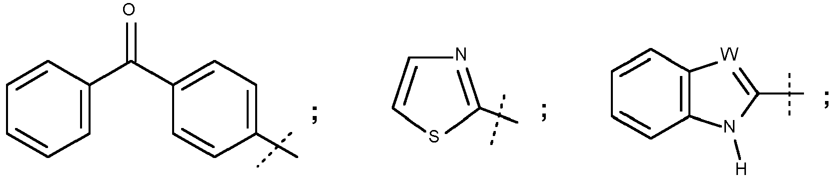 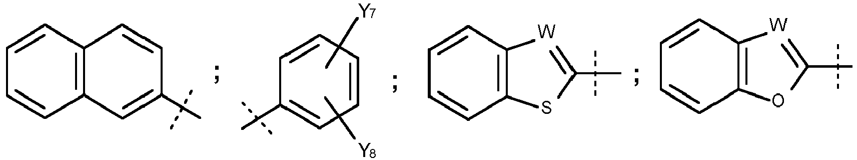 wherein W is selected from CH, O and N, or R is absent,
wherein each of Y₁, Y₂, Y₃, Y₄, Y₅, Y₆, Y₇ and Y₈ is independently selected from the group consisting of H, OH, O-(C₁-C₃)alkyl, C₁-C₃alkyl, NH₂, NH-(C₁-C₃)alkyl, halogen, SO₃H and COOH,
with the proviso that, when each one of R, L, L₁, Ar, Ar₁ is absent and the group on the right of the absent group remains with an open bond, this bond bears a H atom,
the enantiomers, diastereoisomers, and mixtures thereof,
or a pharmaceutically acceptable salt, hydrate or solvate thereof,
for use in the treatment of a disease which can be improved or prevented by an anti-heparanase activity,
with the proviso that said compound of formula (I) is not 1,3-bis-[4-(1H-benzoimidazol-2-yl)-phenyl]-urea.

In a preferred embodiment, said disease is selected from the group consisting of tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular in case of peritoneal fibrosis glucose induced following peritoneal dialysis) and aging, with the proviso that when said compound of formula (I) is 2-Naphthalenesulfonic acid, 7,7'-(carbonyldiimino)bis[4-hydroxy-3-[2-[2-sulfo-4-[2-(4-sulfophenyl)diazenyl]phenyl]diazenyl]- (SST0595) or 2-Naphthalenesulfonic acid, 5,5'-[carbonylbis[imino(2-sulfo-4,1-phenylene)-2,1-diazenediyl]]bis[6-amino-4-hydroxy- (SST0753), said disease is not a tumor and is not metastasis.

Preferably, in the compound of formula (I) both Ar and Ar₁ are present.

Preferably, in the compound of formula (I) both Ar and R or Ar₁ and R are present.

Preferably, Ar, Ar₁ and R are not absent.

In a first preferred embodiment, compounds for the use according to the invention are those in which X is NHCONH and one of Ar and Ar₁ is naphthyl, more preferably substituted with at least one group SO₃H. In this embodiment, preferably at least one of L and L₁ is N=N.

In a second preferred embodiment, compounds for the use according to the invention are those in which X is O and at least one of Ar and Ar₁ is phenyl, preferably they are both present and they are both phenyl.

In a third preferred embodiment, compounds for the use according to the invention are those in which X is C(CH₃)₂, Ar is phenyl, L is O, Ar₁ is phthalimide, L₁ is absent and R is phenyl, preferably substituted with COOH.

In a fourth preferred embodiment, compounds for the use according to the invention are those in which X is 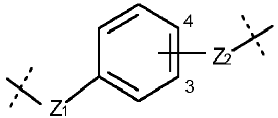 wherein Z₁ and Z₂ are both CONH and Ar is phenyl. In this embodiment, Ar₁ is preferably phthalimide or R is preferably benzimidazole.

### Detailed description of the invention

### Definitions

Within the meaning of the present invention, the terms "alkyl" and "alkylene" refer to linear or branched alkyl and alkylene groups having from 1 to 4 carbon atoms, preferably from 1 to 3 carbon atoms.

Within the meaning of the present invention, the term "anti-heparanase activity" refers to an activity sufficient to inhibit partially or totally the enzyme heparanase.

Within the meaning of the present invention, the dotted lines in the above structures indicate a bond with an adjacent group, as evident for a skilled person. For example, the following chemical structure 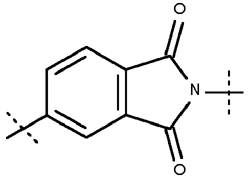 means that the phthalimide group is bound with one adjacent group through the imido function and with another adjacent group through the indicated position 5 on the phenyl ring. In case there is no adjacent group the bond is with an hydrogen.

According to the present invention, a C₁-C₄alkylene group can be for example methylene, ethylene, propylene, 1,1-dimethylmethylene, butylene, or isomers thereof. It can be linear or branched. Preferably, it is 1,1-dimethylmethylene.

According to the present invention, a C₁-C₄alkyl, linear or branched, can be for example methyl, ethyl, n-propyl, isopropyl, butyl, *sec*-butyl, isobutyl, *tert*-butyl.

When a halogen is present, it is selected from the group consisting of: fluorine (F), chlorine (CI), bromine (Br) and iodine (I).

In some embodiments, R is selected from benzimidazole, benzoxazole and benzothiazole.

Preferably Z₁ and Z₂ have the same meaning. Preferably, they are selected from O and CONH.

When Ar or Ar₁ is naphthyl, it can be substituted by one, two, three or four groups according to the definitions of Y₃-Y₆ disclosed above. Preferably, it is substituted on two or three positions. Substituent groups can be on any free position of the ring. Preferred substituents are: SO₃H, OH and NH₂. In an embodiment, Ar or Ar₁ is a naphthyl substituted with one or two groups SO₃H and one group OH. In another embodiment, it is a naphthyl substituted with SO₃H, OH and NH₂.

When Ar or Ar₁ is phenyl, it can be substituted on one or two positions according to the definitions of Y₁-Y₂ disclosed above. Preferred substituents are: SO₃H, CH₃, COOH, OH, halogen and OCH₃. Substituent groups can be on any free position of the ring.

When R is phenyl, it can be substituted on one or two positions according to the definitions of Y₇-Y₈ disclosed above. Preferred substituents are: OH, COOH, SO₃H, F and NH₂. Substituent groups can be on any free position of the ring

In an embodiment, compounds for the use according to the present invention are those wherein X is NHCONH.

In this embodiment, Ar is preferably phenyl or naphthyl.

In this embodiment, one of Ar and Ar₁ is naphthyl, more preferably substituted with at least one group SO₃H.

In a preferred embodiment wherein X is NHCONH, Ar is phenyl, preferably substituted with one or two of the followings groups: SO₃H, OCH₃ and CH₃, L is N=N or NHCO, and Ar₁, L₁ and R have the meanings above defined. In a more preferred embodiment, Ar₁ is naphthyl, preferably substituted with one or more substituents selected from the group consisting of SO₃H, NH₂ and OH, and L₁ and R have the meanings above defined or they are absent.

In another embodiment wherein X is NHCONH, Ar is naphthyl, optionally substituted, for example with one or more SO₃H or OH groups, L is N=N and Ar₁ is phenyl, optionally substituted with one of the meanings above disclosed, and L₁ and R have the meanings above defined. In a preferred embodiment, L₁ is N=N and R is phenyl, optionally substituted, for example with SO₃H.

In another embodiment, compounds for the use according to the present invention are those wherein X is O.

In this embodiment, Ar is preferably phenyl.

In a preferred embodiment wherein X is O, Ar is phenyl, L is CONH, Ar₁ is phenyl, optionally substituted, for example with one or two groups selected among the followings: COOH, OH and a halogen, and L₁ and R have the meanings above defined. In this embodiment, L₁ is preferably absent and R is preferably selected from the group consisting of COOH, benzimidazole, benzoxazole and benzothiazole. In another embodiment, L₁ is CO and R is phenyl.

In another preferred embodiment wherein X is O and Ar is phenyl, L is S and Ar₁, L₁ and R have the meanings above defined. In this embodiment, Ar₁ is preferably phenyl, L₁ is preferably NHCO and R is preferably CH=CHCOOH.

In another preferred embodiment wherein X is O, Ar is phenyl, Ar₁ is thiazole and L, L₁ and R have the meanings above defined. In this embodiment, preferably L is CONH and L₁ is absent or L is absent and L₁ is NHCO. In this embodiment, R is preferably selected from the group consisting of naphthalene, benzophenone and phenyl, optionally substituted with an halogen, such as F.

In another preferred embodiment wherein X is O, one of Ar and Ar₁ is phthalimide.

In particular, in a preferred embodiment wherein X is O and Ar is phenyl, Ar₁ is phthalimide and L, L₁ and R have the meanings above defined. In this embodiment, L is preferably absent, L₁ is preferably O or CONH and R is preferably phenyl, optionally substituted, for example with COOH. In another embodiment, L is NHCO, L₁ is absent and R is thiazole.

In another preferred embodiment wherein X is O, Ar is phthalimide, Ar₁ is phenyl, and L, L₁ and R have the meanings above defined. In this embodiment, L is preferably absent, L₁ is preferably O or is absent and R is preferably phenyl, optionally substituted, for example with one or more COOH groups, or is absent.

In another embodiment, compounds for the use according to the present invention are those in which X is SO₂.

In a preferred embodiment, X is SO₂ and Ar is phenyl and Ar₁, L, L₁ and R have the meanings above defined. In this embodiment, preferably one of L and L₁ is CONH or NHCO and the other one is absent. For example, in an embodiment L is absent, Ar₁ is preferably phthalimide, L₁ is CONH and R is preferably phenyl, optionally substituted, for example with COOH. In an another embodiment, L is CONH, Ar₁ is preferably phenyl, optionally substituted, for example with OH, L₁ is absent and R is preferably COOH. In another embodiment, L is O, Ar₁ is phenyl, L₁ is NHCO and R is phenyl, optionally substituted for example with an halogen, such as F.

In another embodiment, compounds for the use according to the present invention are those in which X is C₁-C₄alkylene, linear or branched. Preferably, it is C(CH₃)₂.

In a preferred embodiment, X is C(CH₃)₂, Ar is phenyl, L is O, Ar₁ is phthalimide, L₁ is absent and R is phenyl, preferably substituted with COOH in position 3 or 4.

In another embodiment, compounds for the use according to the present invention are those in which X is 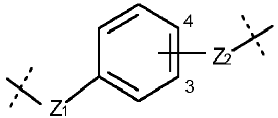 wherein Z₁ and Z₂ have the meanings above defined.

In a preferred embodiment, Z₁ and Z₂ are the same and they are selected from the group consisting of O and CONH.

When Z₁ and Z₂ are both CONH, X has, for example, the following structure 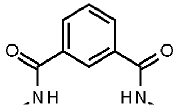 when Z₂ is in position 3 of the phenyl ring.

In a more preferred embodiment, Z₁ and Z₂ are both O or both CONH, Ar is phenyl, optionally substituted, L is absent, Ar₁ is phthalimide, L₁ is absent and R is COOH.

In an another embodiment, Z₁ and Z₂ are both CONH, Ar is phenyl, optionally substituted, for example with a halogen, and Ar₁, L, L₁ and R have the meanings above defined. In this embodiment, L is preferably O or is absent, Ar₁ is preferably phenyl, optionally substituted, for example with COOH, or is phthalimide, L₁ is preferably absent and R is preferably COOH or benzimidazole.

For example, in an embodiment wherein Z₁ and Z₂ are both CONH and Ar is phenyl, optionally substituted, L is preferably absent, Ar₁ is preferably phthalimide, L₁ is absent and R is preferably COOH.

In an another embodiment, Z₁ and Z₂ are both O, Ar and Ar₁ are different and they are chosen from phenyl and phthalimide, L and L₁ are preferably absent, and R is preferably COOH.

When one or more chiral centers are present in the compounds of the present invention, the individual isomers, enantiomers and diastereoisomers, and mixtures thereof (e.g., racemates) are intended to be encompassed by the present invention.

The pharmaceutical acceptable salts of the compound of formula (I) are included in the scope of the invention.

Pharmaceutical acceptable salts are salts which retain the biological activity of the compound and are derived from known pharmacologically acceptable basis. Examples of pharmaceutically acceptable salts are, for example, those from compounds of formula (I) and inorganic bases, such as sodium, potassium, calcium and aluminum hydroxides, as well as those with organic bases such as, for example, lysine, arginine, N-methyl-glucamine, trimethylamine triethanolamine, dibenzylamine, methylbenzylamine, di-(2-ethyl-hexyl)-amine, piperidine, N-ethylpiperidine, N,N-di-ethylaminoethylamine, N-ethylmorpholine, 3-phenethylamine, N-benzyl-3-phenethylamine, N-benzyl-N,N-dimethylamine. A preferred salt is sodium salt.

Also, pharmaceutically acceptable hydrates or solvates of the compound of formula (I) are included in the scope of the present invention. In particular, they can be any hydrate or solvate commonly used in the art.

Exemplary compounds for the use according to the present invention are those listed in the following Table 1. Such compounds are all known in the art and each compound is identified with the respective CAS (Chemical Abstract Service) number.

**Table 1. List of compounds for use in the present invention.**

| **No.** | **Compound Id** | **Name and structural Formula** | **CAS number** |
|---|---|---|---|
| **1** | SST0595NA1 | 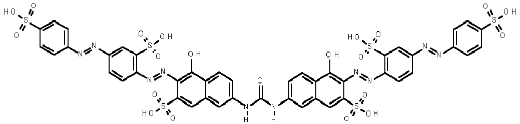 | 25188-41-4 (acid) |
| | | 2-Naphthalenesulfonic acid, 7,7'-(carbonyldiimino)bis[4-hydroxy-3-[2-[2-sulfo-4-[2-(4-sulfophenyl)diazenyl] phenyl]diazenyl]- | 2610-10-8 (sodium salt) |
| **2** | SST0597NA1 | 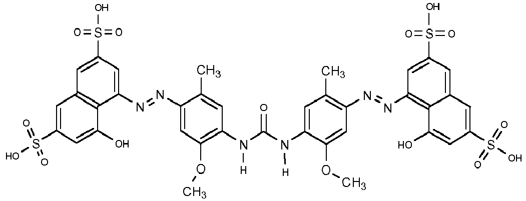 | 25784-17-2 (acid) |
| | | 2,7-Naphthalenedisulfonic acid, 4,4'-[carbonylbis[imino(5-methoxy-2-methyl-4,1-phenylene)azo]]bis[5-hydroxy- | 1937-34-4 (sodium salt) |
| **3** | SST0600AA1 | 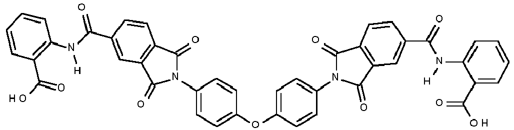 | 294666-29-8 |
| | | Benzoic acid, 2,2'-[oxybis[4,1-phenylene(1,3-dihydro-1,3-dioxo-2H-isoindole-2,5-diyl)carbonylimino]]bis- | |
| **4** | SST0602AA1 | 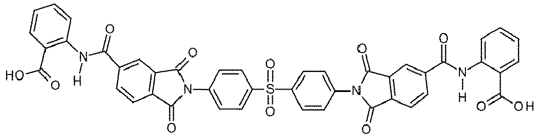 | 352644-81-6 |
| | | 2-[[2-[4-[4-[5-[(2-carboxyphenyl)carbamoyl]-1,3-dioxoisoindol-2-yl]phenyl]sulfonylphenyl]-1,3-dioxoisoindole-5-carbonyl]amino]benzoic acid | |
| **5** | SST0604AA1 | 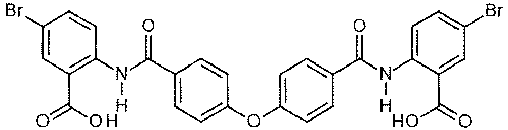 | 330634-98-5 |
| | | 5-bromo-2-[[4-[4-[(4-bromo-2-carboxyphenyl) carbamoyl]phenoxy]benzoyl]amino]benzoic acid | |
| **6** | SST0610NA1 | 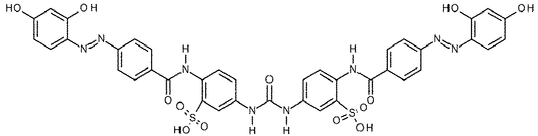 | 789426-28-4 (acid) |
| | | Benzenesulfonic acid, 3,3'-(carbonyldiimino)bis[6-[[4-[(2,4-dihydroxyphenyl)azo]benzoyl]amino]- | 6655-87-4 (sodium salt) |
| **7** | SST0656AA1 | 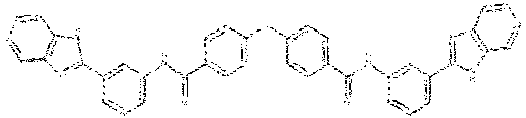 | 477493-81-5 |
| | | N-[3-(1H-benzimidazol-2-yl)phenyl]-4-[4-[[3-(1H-benzimidazol-2-yl)phenyl]carbamoyl]phenoxy]benzamide | |
| **8** | SST0753AA1 | 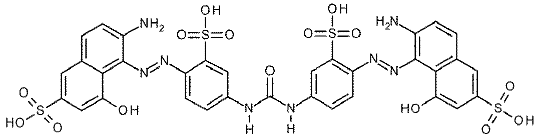 | 70253-90-6 (acid) |
| | | 2-Naphthalenesulfonic acid, 5,5'-[carbonylbis[imino(2-sulfo-4,1-phenylene)-2,1-diazenediyl]]bis[6-amino-4-hydroxy- | 2829-43-8 (sodium salt) |
| **9** | SST0802AA1 | 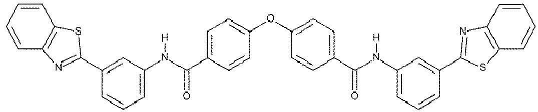 | 476634-38-5 |
| | | N-[3-(1,3-benzothiazol-2-yl)phenyl]-4-[4-[[3-(1,3-benzothiazol-2-yl)phenyl]carbamoyl]phenoxy]benzamide | |
| **10** | SST0805AA1 | 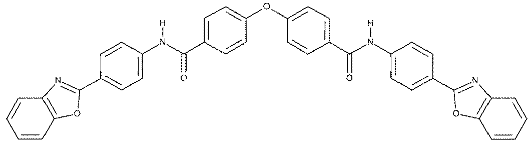 | 351893-10-2 |
| | | Benzamide, 4,4'-oxybis[N-[4-(2-benzoxazolyl) phenyl]- | |
| **11** | SST0806AA1 | 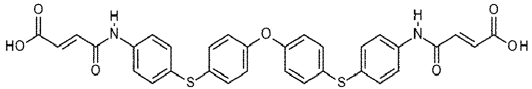 | 351188-09-5 |
| | | (E)-4-[4-[4-[4-[4-[[(E)-3-carboxyprop-2-enoyl]amino]phenyl] sulfanylphenoxy]phenyl]sulfanylanilino]-4-oxobut-2-enoic acid | |
| **12** | SST0807AA1 | 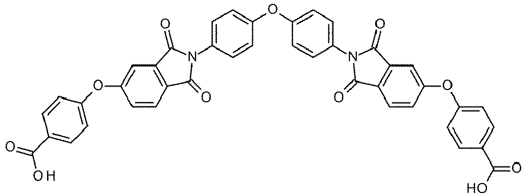 | 148878-10-8 |
| | | 4-[2-[4-[4-[5-(4-carboxyphenoxy)-1,3-dioxoisoindol-2-yl]phenoxy]phenyl]-1,3-dioxoisoindol-5-yl]oxybenzoic acid | |
| **13** | SST0808AA1 | 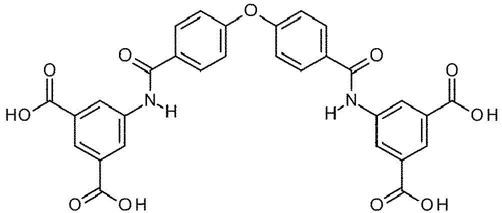 | 304885-58-3 |
| | | 5-[[4-[4-[(3,5-dicarboxyphenyl)carbamoyl]phenoxy]benzoyl] amino]benzene-1,3-dicarboxylic acid | |
| **14** | SST0809AA1 | 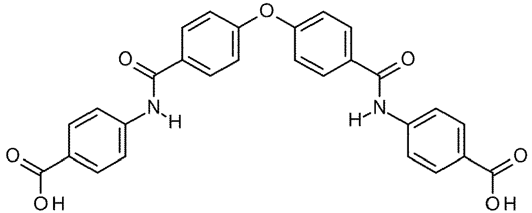 | 304689-04-1 |
| | | 4,4'-[oxybis(benzene-4,1-diylcarbonylimino)]dibenzoic acid | |
| **15** | SST0810AA1 | 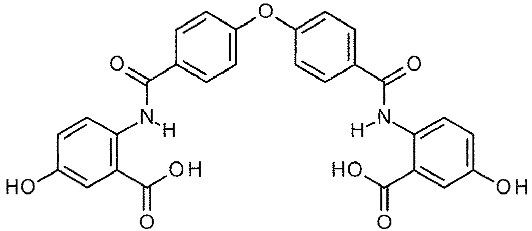 | 327996-87-2 |
| | | Benzoic acid, 2,2'-[oxybis(4,1-phenylenecarbonylimino)]bis[5-hydroxy- | |
| **16** | SST0812AA1 | 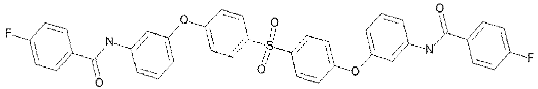 | 330635-99-9 |
| | | Benzamide, N,N'-[sulfonylbis(4,1-phenyleneoxy-3, | |
| | | 1-phenylene)]bis[4-fluoro- | |
| **17** | SST0813AA1 | 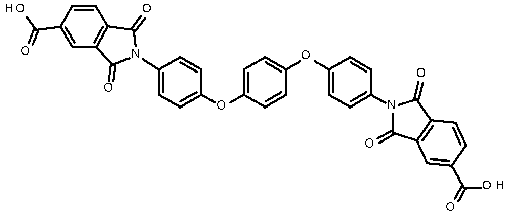 | 130651-50-2 |
| | | 1H-Isoindole-5-carboxylic acid, 2,2'-[1,4-phenylenebis(oxy-4,1-phenylene)]bis[2,3-dihydro-1,3-dioxo- | |
| **18** | SST0814AA1 | 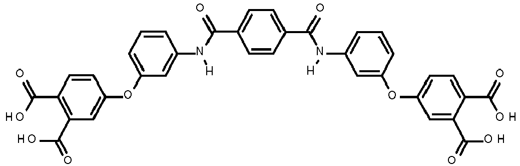 | 330971-85-2 |
| | | 4-[3-[[4-[[3-(3,4-dicarboxyphenoxy)phenyl]carbamoyl] benzoyl]amino]phenoxy]phthalic acid | |
| **19** | SST0817AA1 | 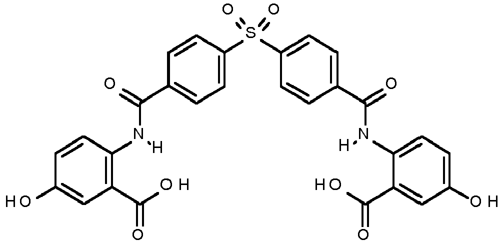 | 609821-77-4 |
| | | Benzoic acid, 2,2'-[sulfonylbis(4,1-phenylenecarbonylimino)] bis[5-hydroxy | |
| **20** | SST0851AA1 | 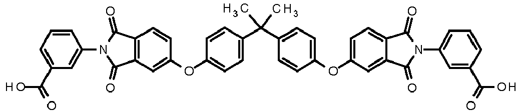 | 226415-59-4 |
| | | 3-[5-[4-[2-[4-[2-(3-carboxyphenyl)-1,3-dioxoisoindol-5-yl]oxyphenyl]propan-2-yl]phenoxy]-1,3-dioxoisoindol-2-yl]benzoic acid | |
| **21** | SST0852AA1 | 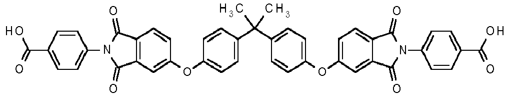 | 134118-32-4 |
| | | 4-[5-[4-[2-[4-[2-(4-carboxyphenyl)-1,3-dioxoisoindol-5-yl]oxyphenyl]propan-2-yl]phenoxy]-1,3-dioxoisoindol-2-yl]benzoic acid | |
| **22** | SST0853AA1 | 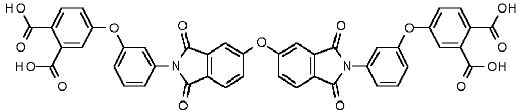 | 351521-52-3 |
| | | 4-[3-[5-[2-[3-(3,4-dicarboxyphenoxy)phenyl]-1,3-dioxoisoindol-5-yl]oxy-1,3-dioxoisoindol-2-yl]phenoxy]phthalic acid | |
| **23** | SST0855AA1 | 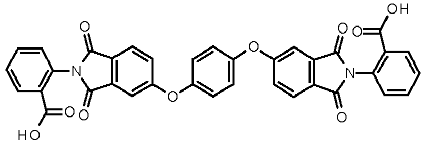 | 302926-71-2 |
| | | Benzoic acid, 2,2'-[1,4-phenylenebis[oxy(1,3-dihydro-1,3-dioxo-2H-isoindole-5,2-diyl)]]bis- | |
| **24** | SST0856AA1 | 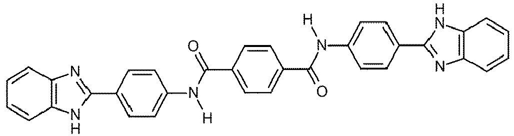 | 191660-01-2 |
| | | 1,4-Benzenedicarboxamide, N1,N4-bis[4-(1H-benzimidazol-2-yl)phenyl]- | |
| **25** | SST0857AA1 | 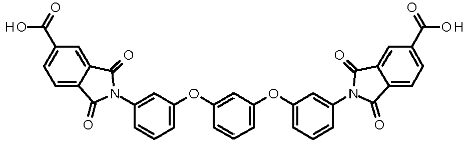 | 401603-58-5 |
| | | 1H-Isoindole-5-carboxylic acid, 2,2'-[1,3-phenylenebis(oxy-3,1-phenylene)]bis[2,3-dihydro-1,3-dioxo- | |
| **26** | SST0859AA1 | 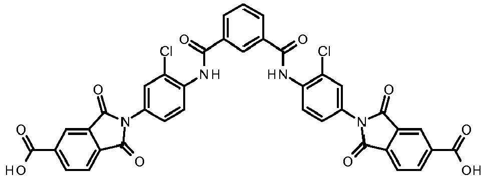 | 340183-01-9 |
| | | 2-[4-[[3-[[4-(5-carboxy-1,3-dioxoisoindol-2-yl)-2-chlorophenyl]carbamoyl]benzoyl]amino]-3-chlorophenyl]-1,3-dioxoisoindole-5-carboxylic acid | |
| **27** | SST0860AA1 | 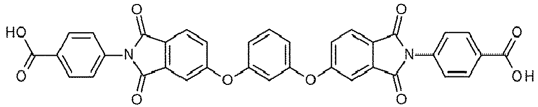 | 330816-92-7 |
| | | Benzoic acid, 4,4'-[1,3-phenylenebis[oxy(1,3-dihydro-1,3-dioxo-2H-isoindole-5,2-diyl)]]bis- | |
| **28** | SST0801AA1 | 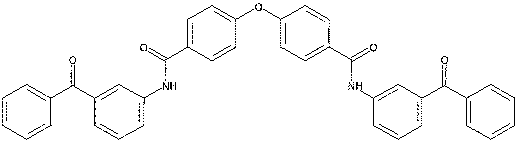 | 351188-37-9 |
| | | Benzamide, 4,4'-oxybis[N-(3-benzoylphenyl)- | |
| **29** | SST0803AA1 | 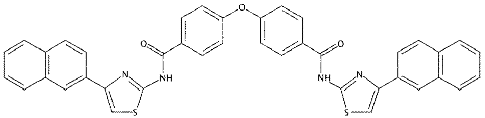 | 476296-13-6 |
| | | Benzamide, 4,4'-oxybis[N-[4-(2-naphthalenyl)-2-thiazolyl]- | |
| **30** | SST0804AA1 | 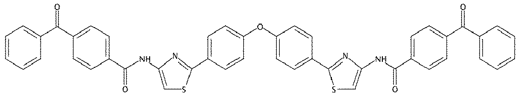 | 397288-95-8 |
| | | Benzamide, N,N'-[oxybis(4,1-phenylene-2,4-thiazolediyl)]bis[4-benzoyl- | |
| **31** | SST0815AA1 | 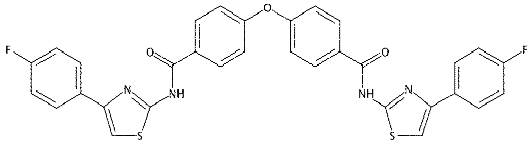 | 476296-10-3 |
| | | Benzamide, 4,4'-oxybis[N-[4-(4-fluorophenyl)-2-thiazolyl]- | |
| **32** | SST0816AA1 | 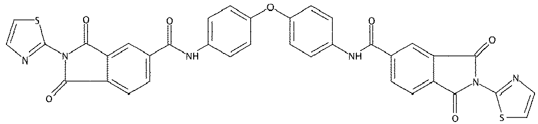 | 295348-44-6 |
| | | 1H-Isoindole-5-carboxamide, N,N'-(oxydi-4,1-phenylene)bis[2,3-dihydro-1,3-dioxo-2-(2-thiazolyl)- | |

Among the above listed compounds, preferred compounds for the use according to the present invention are represented in the following Table 2:

**Table 2. Preferred compounds for use in the present invention.**

| **No.** | **Compound Id** | **Structural Formula** |
|---|---|---|
| **1** | SST0595NA1 | 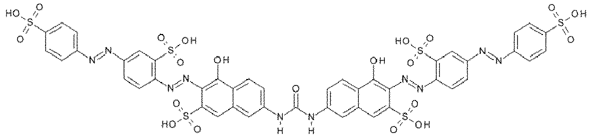 |
| **2** | SST0597NA1 | 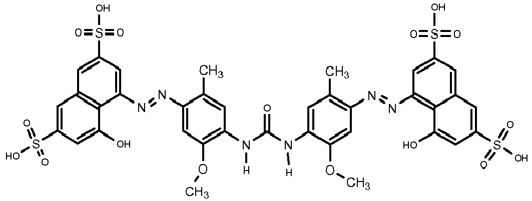 |
| **7** | SST0656AA1 | 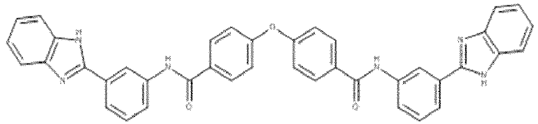 |
| **11** | SST0806AA1 | 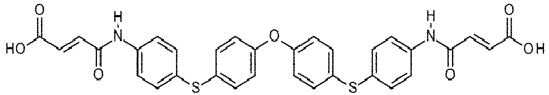 |
| **20** | SST0851AA1 | 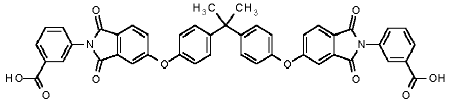 |
| **22** | SST0853AA1 | 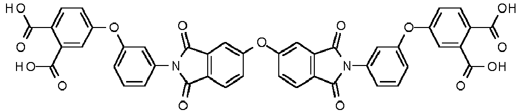 |
| **24** | SST0856 | 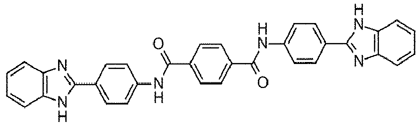 |
| **26** | SST0859 | 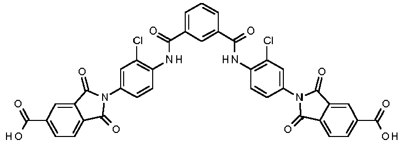 |

Compounds for the use according to the present invention are known.

Therefore, they can be synthesized by many methods known in the art for their synthesis and available to those skilled in the art of organic chemistry. In particular, for the compounds listed in the above Table 1 reference can be made to the respective CAS number wherein exemplary synthesis methods can be found. Different methods or variations of the known methods to prepare the compounds will be evident to those skilled in the art. Also, the skilled in the art can easily slightly alter the reagents and reaction conditions indicated by the state of the art to include any combination of substituents defined in formula (I) and not explicitly represented in the above exemplary compounds according to the general knowledge in the field. Reference can be made, for example, to the handbook "March's Advanced Organic Chemistry, Reactions, Mechanism and structures.", Michael B. Smith, Jerry March, last edition.

### Medical uses

According to the present invention, the compounds of formula (I) are endowed with anti-heparanase activity. Therefore, they can be advantageously used for the treatment of diseases which can be improved or prevented by the inhibition of heparanase activity.

The heparanase has a critical role in modulating the microenvironment via the ECM (extracellular matrix) remodeling and thus influencing a multitude of both normal and disease-related processes, as inflammation, blood coagulation, wound healing, angiogenesis, fibrosis and tumor cell growth, invasion and metastasis (for a review of the roles and activities of heparanase see for example "Heparanase: From basic research to therapeutic applications in cancer and inflammation", Vlodavsky I., Singh P., Boyango I., Gutter-Kapon L., Elkin M., Sanderson RD, Ilan N., Drug Resist Updat. 2016 Nov;29:54-75). Heparanase has also been recently reported to play a fundamental role in many other pathologies, such as nephropathies of different origin, severe infective disorders, gastrointestinal diseases, osteolysis in bone metastases as well as in other bone tissue related pathologies, atherosclerosis, cardiovascular disorders and cutaneous aging. The panorama of diseases affected by heparanase expression and activity also includes the so-called rare diseases, such as amyloidosis, hereditary multiple exostoses and idiopathic avascular necrosis of bone as well as pathologies that are not rare in terms of incidence, but are rarely diagnosed, such as vulvodynia (Rivara et al., 2016).

Therefore, the compounds of formula (I) can be advantageously used in a broad range of diseases.

In particular, heparanase expression is enhanced in cancers, including various carcinomas, sarcomas and hematological malignancies, and it has been demonstrated that it correlates with increased tumor size, tumor angiogenesis, enhanced metastasis and poor prognosis. Indeed, treatments of tumor-bearing mice with heparanase-inhibiting compounds has been shown to markedly attenuate tumor progression further supporting an anti-heparanase therapy for multiple types of cancer (Vlodavski et al., 2016).

Therefore, the compounds of the invention, thanks to their demonstrated activity as heparanase inhibitors, can be advantageously used in the treatment of tumors and metastasis.

In particular, they can be used in the treatment of any kind of tumor, both solid and liquid. For example they can be used in the treatment of solid tumors, such as for example glioma and sarcomas, such as fibrosarcoma and osteosarcoma, and in the treatment of hematological malignancies.

The involvement of heparanase in inflammatory reactions has also been shown (Vlodavski et al., 2016) thus supporting the use of inhibitors of heparanase also in diseases characterized by or related with acute and chronic inflammation, such as colitis, atherosclerosis, pancreatitis, Chron's disease, psoriasis and others.

More recently, heparanase overexpression has been documented to be related to the endothelial mesenchymal transition (EMT; Masola V et al. Specific heparanase inhibition reverses glucose-induced mesothelial-to-mesenchymal transition Nephrol Dial Transplant 2017; 1-9) and thus heparanase inhibition may be also beneficial in those pathologic processes involving EMT, as fibrosis occurring at different organs.

Examples of diseases which can be treated by compounds of formula (I) are tumors, primary tumours, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis, in particular if induced by glucose following peritoneal dialysis, and aging.

The skilled in the art, for example a physician, can identify and recognize if a disease can be ameliorated by an anti-heparanase activity on the basis of the common knowledge in the field. For example, for a broad, even though not exhaustive, overview on possible clinical applications of heparanase inhibitors, reference can be made to the reviews of Rivara et al., 2016, and Vlodavski et al., 2016. Tests are known in the field and available to the skilled person to evaluate if a compound is endowed with an anti-heparanase activity, for example the AGAIA assay (Hammond E. et al. Anal. Biochem. 2010, 396, 112).

In a preferred embodiment, compounds of formula (I) are used for the treatment of anyone of the following diseases: diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis and aging.

In an embodiment of the present invention, when the compound of formula (I) is 2-Naphthalenesulfonic acid, 7,7'-(carbonyldiimino)bis[4-hydroxy-3-[2-[2-sulfo-4-[2-(4-sulfophenyl)diazenyl]phenyl]diazenyl]- (SST0595) or 2-Naphthalenesulfonic acid, 5,5'-[carbonylbis[imino(2-sulfo-4,1-phenylene)-2,1-diazenediyl]]bis[6-amino-4-hydroxy-(SST0753) the disease is not tumor or metastasis.

In an embodiment of the invention the compound of formula (I) is used as an adjuvant of a chemotherapeutic agent or drug, in particular in the treatment of a tumor disease or metastasis. For adjuvant it is intended an agent that modifies and/or facilitate the effect of another agent. In the present case, it is intended that in some embodiments the compound of formula (I) is administered in concomitance with a chemotherapeutic drug to enhance, improve or facilitate the tumor killing action of the chemotherapeutic drug. In this embodiment, the compound of formula (I) does not directly exercise its action on the tumor but, thanks to its inhibitory action on the heparanase, it modifies the tumor microenvironment and facilitates the activity of the chemotherapeutic drug.

Compounds of the invention are particularly advantageous also for non-tumor applications since they are endowed with a strong anti-heparanase activity and therefore they can already be efficiently used at very low doses at which they are not cytotoxic. Indeed, in some embodiments they are selective heparanase inhibitors already at concentrations in which they do not interfere significantly with cell proliferation. This renders them particularly useful for therapeutic applications wherein a cytotoxic effect is undesired. Also, it has been found that such compounds at not cytotoxic concentrations are already able to inhibit invasion of tumor cells thus being particularly useful in the treatment of tumors and metastasis, also as adjuvants of chemotherapeutic agents. The compounds of the invention are also advantageously able to interfere with adhesion properties of cancer cells and they have an inhibitory effect against tumor transcription genes encoding for proangiogenic factors thus further supporting their therapeutic use in cancer diseases and metastasis.

The skilled person in the field knows how to determine the suitable dose in view of the desired application and his general knowledge in the medical and pharmaceutical field and by carrying out routine experimentation.

Another object of the present invention is a pharmaceutical composition containing at least one compound of formula (I) as an active ingredient, in an amount such as to produce a significant therapeutic effect, together with pharmaceutically acceptable vehicles and/or excipients for use in the treatment of a disease which can be improved or prevented by an anti-heparanase activity. In particular, the above mentioned diseases can be treated by such pharmaceutical compositions.

Such pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

The compositions according to the present invention contain, along with the active ingredient, at least one pharmaceutically acceptable vehicle or excipient. These may be particularly useful formulation coadjuvants, e.g., solubilising agents, dispersing agents, suspension agents, and emulsifying agents.

The pharmaceutical composition according to the invention comprising the compound of formula (I) can also contain one or more further active ingredients, for example chemotherapeutic agents. In particular, when the pharmaceutical composition is used for the treatment of a tumor, it preferably comprises also a chemotherapeutic agent. In fact, heparanase inhibition does not exert a direct cytotoxic effect, but rather modifies the tumor microenvironment to affect cell growth and spread as well as to facilitate the tumor killing action of other agents. This makes possible the combination of the compounds of the invention with any chemotherapeutic agent, which can therefore be chosen among the known agents according to the general knowledge of the skilled person in the field. For example, said chemotherapeutic agent can be selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens. When said chemotherapeutic agent is a cytotoxic agent, it is preferably selected from the group consisting of melphalan, irinotecan, rituximab and lapatinib. When said chemotherapeutic agent is a proteasome inhibitor, it is preferably selected from the group consisting of bortezomib, carfilzomib and ixazomib. When said chemotherapeutic agent is an immunomodulatory agent, it is preferably selected from the group consisting of thalidomide, lenalidomide and pomalidomide.

For example, when the disease to be treated is multiple myeloma (MM), the compounds of the invention can be administered in conjunction with chemotherapy drugs known to be effective in MM, such as for example cytotoxic agents, for example melphalan, proteasome inhibitors, immunomodulatory drugs (IMIDs) and monoclonal antibodies towards highly expressed myeloma associated antigens.

In an embodiment of the invention, such pharmaceutical composition comprises 2-Naphthalenesulfonic acid, 7,7'-(carbonyldiimino)bis[4-hydroxy-3-[2-[2-sulfo-4-[2-(4-sulfophenyl)diazenyl]phenyl]diazenyl]- (SST0595) or 2-Naphthalenesulfonic acid, 5,5'-[carbonylbis[imino(2-sulfo-4,1-phenylene)-2,1-diazenediyl]]bis[6-amino-4-hydroxy-(SST0753) as compound of formula (I) and a chemotherapeutic agent wherein the compound of formula (I) is used as an adjuvant to facilitate the action of said chemotherapeutic agent, in particular in the treatment of a tumor disease or a metastasis.

According to the administration route chosen, the compositions will be in solid or liquid form, suitable for oral, parenteral or topical administration.

Generally, the compounds of this invention are administered in a "therapeutically effective amount". The amount of the compound actually administered will typically be determined by a physician, in the light of the relevant circumstances, including the condition to be treated, the chosen route of administration, the actual compound administered, drug combination, the age, body weight, and response of the individual patient, the severity of the patient's symptoms, and the like. For any compound, the therapeutically effective dose can be estimated initially either in cell culture assays or in animal models, usually mice, rats, guinea pigs, rabbits, dogs, or pigs. The animal model may also be used to determine the appropriate concentration range and route of administration. Such information can then be used to determine useful doses and routes for administration in humans. In calculating the Human Equivalent Dose (HED) it is recommended to use the conversion table provided in Guidance for Industry and Reviewers document (2002, U.S. Food and Drug Administration, Rockville, Maryland, USA).

Generally, an effective dose will be from 0.01 mg/kg to 100 mg/kg, preferably 0.05 mg/kg to 50 mg/kg. For any compound, the therapeutically effective dose can be estimated initially either in cell culture assays or in animal models, usually mice, rats, guinea pigs, rabbits, dogs, or pigs. The precise effective dose for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

Compositions may be administered individually to a patient or may be administered in combination with other agents, drugs or hormones. For example they can be used in combination with further active ingredients, such as for example anticancer drugs.

The medicament may also contain a pharmaceutically acceptable carrier, for administration of a therapeutic agent. Such carriers include antibodies and other polypeptides, genes and other therapeutic agents such as liposomes, provided that the carrier does not induce the production of antibodies harmful to the individual receiving the composition, and which may be administered without undue toxicity.

Suitable carriers may be large, slowly metabolised macromolecules such as proteins, polysaccharides, polylactic acids, polyglycolic acids, polymeric amino acids, amino acid copolymers and inactive virus particles.

A thorough discussion of pharmaceutically acceptable carriers is available in Remington's Pharmaceutical Sciences (Mack Pub. Co., N.J. 1991).

Pharmaceutically acceptable carriers in therapeutic compositions may additionally contain liquids such as water, saline, glycerol and ethanol.

Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The medicament of this invention may be administered by any number of routes including, but not limited to, oral, intravenous, intramuscular, intra-arterial, intramedullary, intrathecal, intraventricular, transdermal or transcutaneous applications, subcutaneous, intraperitoneal, intranasal, enteral, topical, sublingual, intravaginal or rectal means.

The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include refilled, pre-measured ampoules or syringes of the liquid compositions or pills, tablets, capsules or the like in the case of solid compositions. In such compositions, the compound of the invention is usually a minor component (from about 0.1 to about 50% by weight or preferably from about 1 to about 40% by weight) with the remainder being various vehicles or carriers and processing aids helpful for forming the desired dosing form. Dosage treatment may be a single dose schedule or a multiple dose schedule.

Pharmaceutical compositions for the use of the invention can be obtained by mixing one or more compounds of formula (I) with suitable excipients, stabilizers and/or pharmaceutically acceptable diluents.

Pharmaceutical compositions and preparations thereof are within the general knowledge and reference can be made to conventional manuals and national and European Pharmacopoeias.

In an embodiment, the compounds of the invention can also be used for cosmetic applications thanks to their activity of inhibition of heparanase. Indeed, heparanase is produced in the skin by epidermal keratinocytes composing the epidermis and fibroblasts or vascular endothelial cells of the dermis and it decomposes heparan sulfate proteoglycan, which regulates differentiation and growth of the epidermis. It is known that heparanase can promote the formation of wrinkles; indeed, inhibition of heparanase activity suppresses the release of growth factors that accompanies decomposition of heparan sulfate, and allows migration of growth factors between the epidermis and dermis to be controlled, thereby aiding in anti-aging and anti-wrinkles effects of the skin (see for example EP2484349, US2014080878 and the work "Hyperpigmentation in human solar lentigo is promoted by heparanase-induced loss of heparan sulfate chains at the dermal-epidermal junction." Iriyama S, Ono T, Aoki H, Amano S. Dermatol Sci. 2011 Dec;64(3):223-8).

In view of the above and of the heparanase inhibition activity of the compounds of the invention, it is also an object of the present invention the cosmetic use of the compounds of formula (I) for prevention or treatment of skin aging. In particular, they can be used for preventing or suppressing the formation of wrinkles, in particular wrinkles caused by reduced level of heparanase sulfate in the epidermal basal membrane.

The compounds of the invention are particularly suitable for such cosmetic applications due to their low cytotoxicity. In particular, due to their potent anti-heparanase activity, they can already exert their effect at very low doses, at which they are not cytotoxic and thus suitable for cosmetic uses.

It is also an object of the invention, a cosmetic composition comprising the compound of formula (I) and cosmetically acceptable excipients and/or vehicles for use as heparanase inhibitors in the prevention or suppression of wrinkles or in any other anti-aging applications. Such excipients or vehicles can be chosen by the skilled person among the cosmetic ingredients commonly used in the field. In particular, they are components commonly used in external preparations. Such cosmetic composition is preferably applied and administered topically.

The following examples further illustrate the invention.

### EXAMPLE 1

### In vitro screening for heparanase activity

### Materials and methods

All compounds were tested in a simple, accurate and robust microplate assay based on the cleavage of the synthetic heparin fragment, the pentasaccharide Fondaparinux (AGA*IA). This assay was originally developed by Hammond and coworkers [Hammond E, Li CP, Ferro V. Development of a colorimetric assay for heparanase activity suitable for kinetic analysis and inhibitor screening. Anal. Biochem. 396(1), 112-116 (2010)], where Fondaparinux corresponds to the methyl glycoside of the antithrombin III (ATIII)-activating pentasaccharide sequence of heparin. Fondaparinux is cleaved by heparanase to generate a reducing disaccharide, which can be quantitatively measured via a colorimetric or a fluorescence assay, the last by the fluorescent sensor polymer-H [Schiemann S, Lühn S, Alban S. Development of both colorimetric and fluorescence heparinase activity assays using fondaparinux as substrate. Anal. Biochem. 427(1), 82-89 (2012)].

To determine the activity of the newly designed heparanase inhibitors an homogenous assay based on the cleavage of the synthetic heparin oligosaccharide fondaparinux has been employed (Hammond E. et al. Anal. Biochem. 2010, 396, 112). The assay measures the appearance of the disaccharide product of heparanase-catalyzed fondaparinux cleavage colorimetrically using the tetrazolium salt WST-1. Assay was essentially performed as described with minor modifications. Briefly, NUNC ELISA 96-well plates were pre-treated with a solution of 4% BSA (bovin serum albumin) in phosphate-buffered saline containing 0.05% Tween 20 (PBST), for 2 h at 37°C, then washed three times with PBST, dried by tapping on paper towel and stored at -20°C. Before use, once again, the plates were washed with PBST.

Assay was carried out with 100 µl/well of assay solution containing 40 mM sodium acetate buffer (pH 5.0), 100 µM fondaparinux, 2.5 nM heparanase and serial dilutions of test compounds (tested in triplicate). Plates were sealed with adhesive tape and incubated, in the dark, for 3 h at 37°C, followed by development with 1.69 mM of the tetrazolium salt WST-1 solution in 0.1 M NaOH, for 1 h at 60°C. Then, the absorbance at 560 nm was measured through a microplate reader (Victor 3, Perkin Elmer).

IC50 value for each compound, versus heparanase, was calculated by GraphPad software and results of selected compounds are summarized in the Table below. Finally, the measurements were corrected by subtracting both the reagent background and the inner absorbance value of test compound.

### Results

Results are shown in the following Table 3.

**Table 3. Results on AGA*IA assay of tested compounds.**

| No. | Compound Id | M.W. (Da) | AGAIA assay (IC50) |
|---|---|---|---|
| Reference | Roneparstat SST0001 | *∼* 20**,**000 | *∼* 5 nM |
| Reference | Suramin (SIGMA-S2671) | 1297.28 | ∼ 26 µM |
| 1 | SST0595 | 1241.18 | ++ |
| 2 | SST0597 | 960.94 | +++ |
| 3 | SST0600 | 786.70 | + |
| 4 | SST0602 | 834.76 | + |
| 5 | SST0604 | 654.26 | + |
| 6 | SST0610 | 882.83 | + |
| 7 | SST0656 | 640.69 | +++ |
| 8 | SST0753 | 902.86 | + |
| 9 | SST0802 | 674.79 | + |
| 10 | SST0805 | 642.66 | + |
| 11 | SST0806 | 612.67 | ++ |
| 12 | SST0807 | 732.65 | + |
| 13 | SST0808 | 584.49 | + |
| 14 | SST0809 | 496.47 | + |
| 15 | SST0810 | 528.47 | + |
| 16 | SST0812 | 676.68 | + |
| 17 | SST0813 | 640.55 | + |
| 18 | SST0814 | 676.58 | + |
| 19 | SST0817 | 576.53 | ++ |
| 20 | SST0851 | 758.73 | ++ |
| 21 | SST0852 | 758.73 | + |
| 22 | SST0853 | 820.67 | ++ |
| 23 | SST0855 | 640.55 | + |
| 24 | SST0856 | 548.59 | +++ |
| 25 | SST0857 | 640.55 | + |
| 26 | SST0859 | 763.49 | +++ |
| 27 | SST0860 | 640.55 | + |
| 28 | SST0660 | 756.72 | + |
| 29 | SST0801 | 616.66 | + |
| 30 | SST0803 | 674.79 | + |
| 31 | SST0804 | 782.88 | + |
| 32 | SST0815 | 610.65 | + |
| 33 | SST0816 | 712.71 | + |

| | | | |
|---|---|---|---|
| Legend: + (1.00-10.00 µM) ++ (0.50-0.99 µM) +++ (0.05-0.49 µM) | | | |

All compounds of examples 1-33 showed a strong anti-heparanase activity in the AGA*IA assay, in particular more potent than suramin, the latter being a reference compound for heparanase inhibition activity.

Upon screening for inhibition of heparanase enzymatic activity, selected active compounds were furtherly characterized in vitro, through a suitable cell proliferation assay (SRB Cell Cytotoxicity Assay) for their putative cytotoxic activity against three tumor cell lines, and through specific cellular assay for their putative anti-metastatic activity. According to these data selected compounds were also tested for the effects on real-time quantitative PCR.

### Example 2

### Cytotoxicity assays

### Materials and methods

SRB Cell Cytotoxicity Assay is an assay based upon the quantitative staining of cellular proteins by sulforhodamine B (SRB). This assay can be used for high-throughput drug screening (Vichai & Kirkitara, Nature Protocol. 2006, 1, 112). Sulforhodamine B is an anionic aminoxanthene dye that forms an electrostatic complex with the basic amino acid residues of proteins under moderately acid conditions, which provides a sensitive linear response.

To assess a putative antiproliferative activity of the selected compounds, HT1080 (human fibrosarcoma), U87MG (human glioblastoma astrocytoma) and U2OS (human osteosarcoma) cell lines were treated for 72h, in triplicate, with increasing concentrations of heparanase inhibitors. Inhibition of cell proliferation was then measured by the classical colorimetric SRB Cell Cytotoxicity assay. Absorbance was measured by spectrophotometer at a wavelength of 510 nm and cell proliferation was determined as percent with respect to control cells (cells treated with the vehicle alone). EC₅₀ values were then determined by GraphPad Prism.

### Results

The following Table 4 reports the concentration of test compounds at which the cell growth is inhibited by 50 % versus control of the tested compounds.

As reference compound Suramin was used.

**Table 4. Putative antiproliferative activity of the selected compounds on three tumor cell lines. Compounds 7, 11, 20, 22, 24, 26 were tested at a maximum concentration of 2.5 µM.**

| | | Concentration of test compounds at which the cell growth is inhibited by 50 % | | |
|---|---|---|---|---|
| No. | Compound Id | **HT1080** | **U87MG** | **U2OS** |
| *Reference compound* | Suramin | > 10 µM | > 10 µM | > 10 µM |
| 1 | SST0595 | > 10 µM | > 10 µM | > 10 µM |
| 2 | SST0597 | > 10 µM | > 10 µM | > 10 µM |
| 7 | SST0656 | > 2.5 µM | > 2.5 µM | > 2.5 µM |
| 11 | SST0806 | > 2.5 µM | > 2.5 µM | > 2.5 µM |
| 20 | SST0851 | > 2.5 µM | > 2.5 µM | > 2.5 µM |
| 22 | SST0853 | > 2.5 µM | > 2.5 µM | > 2.5 µM |
| 24 | SST0856 | > 2.5 µM | > 2.5 µM | > 2.5 µM |
| 26 | SST0859 | > 2.5 µM | > 2.5 µM | > 2.5 µM |

All selected compounds, as well as reference compound (suramin), tested up to 2.5 µM, (10 µM for suramin, SST0595 and SST0597) do not interfere with proliferative activity of the three tumor cell lines. These data are very important because they show that some compounds of the present invention are selective heparanase inhibitors at concentrations in which they do not have any interference with cell proliferation.

Selected compounds were also tested in an invasion assay as well as in cell adhesion assay.

### Example 3

### Invasion and cell adhesion assays

### Materials and methods

### The Invasion Assay

Heparanase inhibitors were then evaluated for their activity on the invasive potential of HT1080 (human fibrosarcoma) human cell lines, using the Matrigel cell invasion assay which allows to assess the cell invasion ability of malignant and normal cells.

Invasion assay was performed using BioCoat cell culture inserts (BioCoat 8.0µm PET Membrane 24 Well Permeable Supports, Corning). Filters were coated with 100 µl of 300 µg/ml Matrigel dissolved in coating buffer (0.01M Tris pH 8.0, 0.7% NaCl) (Becton-Dickinson Sciences). DMEM (Dulbecco's Modified Eagle's Medium) containing 5% FBS was added to the lower chambers. Cells were pre-treated with heparanase inhibitors for 24 h, detached and added to the upper chambers in media containing the inhibitors. Cells were allowed to invade for 24 h at 37°C in a CO₂ incubator and then unmigrated cells were removed from the upper chamber with a cotton swab. The invading cells were fixed, stained with 0.1% crystal violet for 10 min at room temperature, photographed and counted under inverted microscope.

Test compounds were analyzed at fixed concentration for several fields, in duplicates. Activity of test compounds was compared to the reference compound. Activity of most interesting compound was confirmed in a second cell invasion assay. Data were expressed as the percent invasion with respect to cell migration through uncoated insert membrane. (Table 5)

### Cell Adhesion Assay

Cell surface, non-integrin molecules such as heparan sulfate proteoglycans (HSPGs) may be involved in the regulation of early adhesive stages of cell-ECM interactions. A cell adhesion assay has been employed to determine if the newly designed heparanase inhibitors were able to interfere with adhesion properties of cancer cells.

HT1080 (human fibrosarcoma) cells were treated for 18-20 h with test compounds in complete medium supplemented with 10% FCS. Then cells were collected, washed in complete medium, and added to matrigel coated wells in triplicate, at previously identified optimal seeding density, and incubated at 37 °C in complete medium for times ranging from 15 minutes to 4h, according to the cell line. After incubation, the wells were washed three times with serum-free medium and the remaining firmly attached cells were stained with crystal-violet 0.1%. Optical density was measured at 560 nm by Victor 3 (Perkin-Elmer) plate analyzer. Each experiment was repeated three times.

### Results

Results of invasion and adhesion assays are reported in the following Table 5.

**Table 5. Test compounds were analyzed at fixed concentration (2.5 µM) versus Reference compound (Suramin) at 10 µM. In Invasion assay, data were expressed as the percent invasion (range) with respect to cell migration through uncoated insert membrane. In Adhesion assay, data were expressed as the percent of adhesion with respect to the control (100%).**

| No. | Compound Id | Conc. | Invasion Assay Inhibition (%) | | | Adhesion Assay (%) | |
|---|---|---|---|---|---|---|---|
| | | | HT1080 | U87MG | U2OS | HT1080 | U87MG |
| | suramin | 10 µM | + | - | - | ∼ 100 | ∼ 73 |
| 2 | SST0597 | 10 µM | +++ | + | - | ∼ 85 | ∼ 23 |
| 7 | SST0656 | 2.5 µM | + | + | + | ∼ 87 | ∼ 97 |
| 24 | SST0856 | 2.5 µM | + | + | ++ | ∼ 98 | ∼ 99 |
| 26 | SST0859 | 2.5 µM | + | + | ++ | ∼ 95 | ∼ 100 |

| | | | | | | | |
|---|---|---|---|---|---|---|---|
| Legend: +++: > 90% ++: 50-90 % +: < 50% -: Not active | | | | | | | |

The four selected compounds tested at concentration at which they showed limited antiproliferative activity while are active as anti-heparanase, inhibited both invasion and adhesion. Invasion is known to be related to the heparanase activity unlike adhesion that is not directly related to the enzyme inhibition. Therefore, the effect on adhesion should be considered as an adjuvant advantageous effect of the tested compounds.

### Example 4

### Real-Time Quantitative PCR (qPCR) for measuring mRNA gene-expression of FGF1/2, VEGF, MMP-9, HSPE-1 in genes tumor cell lines

### Materials and methods

To evaluate the effect of heparanase inhibitors on the tumor transcription of genes encoding for proangiogenic factors, such as FGF1/2, VEGF, MMP-9, HSPE-1, the relative mRNA levels were analyzed by quantitative Real-Time Quantitative PCR. To perform the assay, HT1080 (human fibrosarcoma) cells were cultured in complete medium containing 10%FCS (fetal calf serum). Cells were plated in 24-well plates and treated at fixed concentration (i.e., highest non-toxic concentration) of test compounds. After 24 h, cells were detached, collected and total RNA extracted by RNeasy Mini Kit (QIAGEN). Following reverse transcription step, FGF1/2, VEGF, MMP-9, HSPE-1 mRNA levels, were quantified by qPCR using the advanced Universal SYBR Green Supermix (Bio Rad) and ABI PRISM 7900HT Thermal Cycler System (Perkin Elmer), according to the manufacturer's instructions. Appropriate no-RT and non-template controls were included in each 96-well PCR reaction, and dissociation analysis was performed at the end of each run to confirm the specificity of the reaction. Relative levels of RNA were calculated using the ddCt method. Standard curve and sample data were run in duplicate. In the Table a mean value is reported.

### Results

Results are shown in the following table 6.

**Table 6. Real-Time RT-PCR of pro-angiogenic genes**

| No. | | | Gene Expression (% mRNA vs. control) | | | | |
|---|---|---|---|---|---|---|---|
| | | Conc | FGF-1 | FGF-2 | VEGF | MMP-9 | HPSE-1 |
| | Control | | ∼ 100 | ∼ 100 | ∼ 100 | ∼ 100 | ∼ 100 |
| | SST0001 | 10.0 uM | ∼ 70 | ∼ 77 | ∼ 43 | ∼ 42 | ∼ 70 |
| **2** | **SST0597** | 10.0 uM | ∼ 100 | ∼ 82 | ∼ 100 | ∼ 69 | ∼ 100 |
| **24** | **SST0856** | 2.5 uM | ∼ 53 | ∼ 76 | ∼ 54 | ∼ 55 | ∼ 76 |
| **26** | **SST0859** | 2.5 uM | ∼ 67 | ∼ 73 | ∼ 61 | ∼ 40 | ∼ 97 |

Data from qPCR assay, with three selected active compounds versus SST0001 (reference compound) on HT1080 (human fibrosarcoma) cells, highlighted an inhibitory effect against the tumor transcription genes encoding for nearly all proangiogenic factors tested.

SST0856AA1 and SST05859AA1, tested at a concentration of 2.5µM, showed a potency in silencing gene expression, in the same range of reference compound SST0001, tested at 10 µM.

## Claims

1. A compound having the following formula (I)
[R-L₁-Ar₁-L-Ar]₂-X (I)
wherein
X is selected from the group consisting of NHCONH, O, SO₂, C₁-C₄alkylene, linear or branched, and 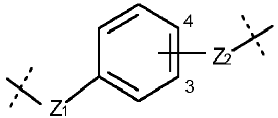 wherein Z₁ and Z₂ are the same or different and each of Z₁ and Z₂ is independently selected from the group consisting of O, S and CONH or it is absent, and Z₂ is in position 3 or 4 of the phenyl ring,
each of Ar and Ar₁ is independently selected from the group consisting of 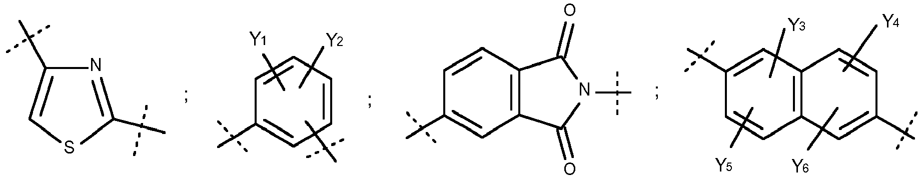
or is absent, with the proviso that at least one of Ar and Ar₁ is present,
each of L and L₁ is independently selected from the group consisting of N=N, NHCO, CONH, CO, S and O, or is absent,
R is selected from the group consisting of COOH, halogen, CH=CHCOOH, 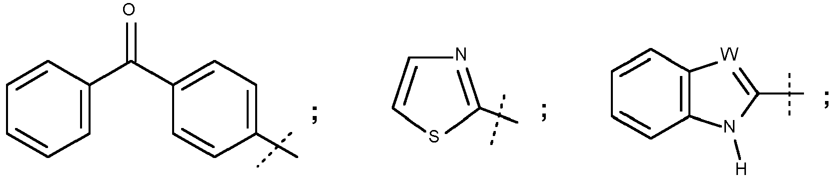 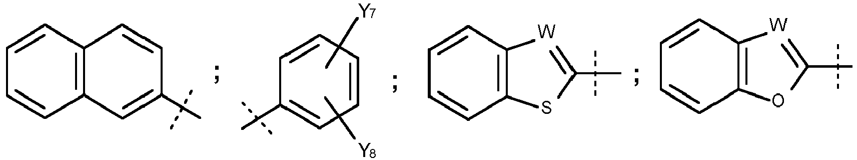 wherein W is selected from CH, O and N, or R is absent,
wherein each of Y₁, Y₂, Y₃, Y₄, Y₅, Y₆, Y₇ and Y₈ is independently selected from the group consisting of H, OH, O-(C₁-C₃)alkyl, C₁-C₃alkyl, NH₂, NH-(C₁-C₃)alkyl, halogen, SO₃H and COOH,
with the proviso that, when each one of R, L, L₁, Ar, Ar₁ is absent and the group on the right of the absent group remains with an open bond, this bond bears a H atom,
the enantiomers, diastereoisomers, and mixtures thereof,
or a pharmaceutically acceptable salt, hydrate or solvate thereof,
for use in the treatment of a disease which can be improved or prevented by an anti-heparanase activity,
with the proviso that said compound of formula (I) is not 1,3-bis-[4-(1H-benzoimidazol-2-yl)-phenyl]-urea.

2. The compound for the use according to claim 1 wherein both Ar and Ar₁ are present.

3. The compound for the use according to claim 1 wherein both Ar and R are present.

4. The compound for the use according to claim 1 wherein both Ar₁ and R are present.

5. The compound for the use according to anyone of claims 1-4 wherein Ar, Ar₁ and R are not absent.

6. The compound for the use according to anyone of claims 1-5 wherein X is 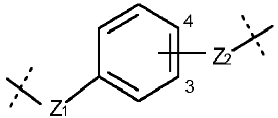 wherein Z₁ and Z₂ are both CONH and Ar is phenyl.

7. The compound for the use according to anyone of claims 1-5 wherein X is NHCONH and one of Ar and Ar₁ is naphthyl, preferably substituted with at least one group SO₃H.

8. The compound for the use according to anyone of claims 1-5, wherein X is O and at least one of Ar and Ar₁ is phenyl, preferably they are both phenyl.

9. The compound for the use according to anyone of claims 1-5 wherein X is C(CH₃)₂, Ar is phenyl, L is O, Ar₁ is phthalimide, L₁ is absent and R is phenyl, preferably substituted with COOH.

10. The compound for the use according to anyone of the preceding claims wherein said compound is one of the followings:
| |
|---|
| 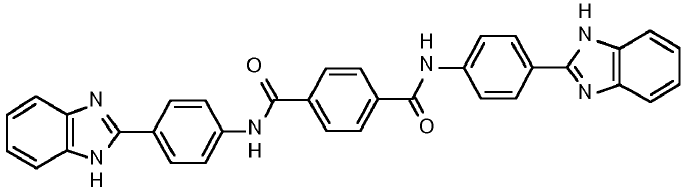 |
| 1,4-Benzenedicarboxamide, N1,N4-bis[4-(1H-benzimidazol-2-yl)phenyl]- |
| 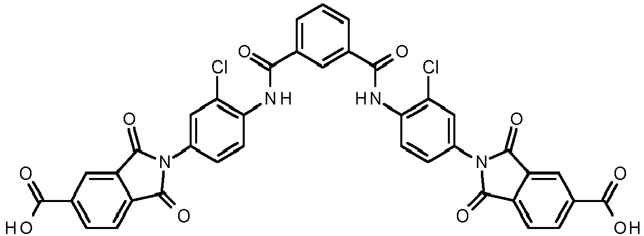 |
| 2-[4-[[3-[[4-(5-carboxy-1,3-dioxoisoindol-2-yl)-2-chlorophenyl]carbamoyl]benzoyl]amino]-3-chlorophenyl]-1,3-dioxoisoindole-5-carboxylic acid |
| 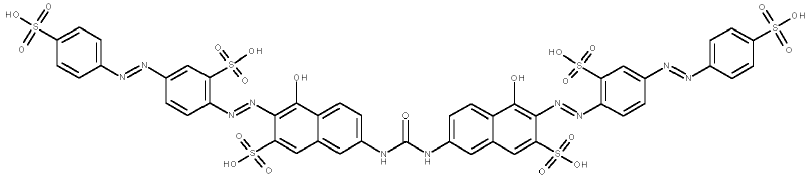 |
| 2-Naphthalenesulfonic acid, 7,7'-(carbonyldiimino)bis[4-hydroxy-3-[2-[2-sulfo-4-[2-(4-sulfophenyl) diazenyl]phenyl]diazenyl]- |
| 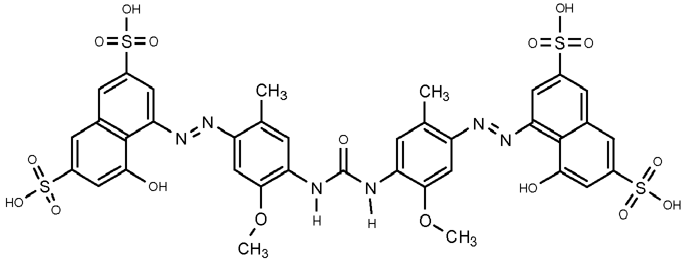 |
| 2,7-Naphthalenedisulfonic acid, 4,4'-[carbonylbis[imino(5-methoxy-2-methyl-4,1-phenylene)azo]]bis[5-hydroxy- |
| 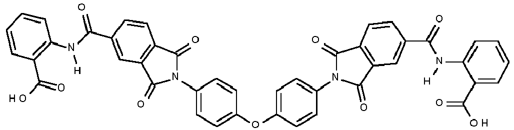 |
| Benzoic acid, 2,2'-[oxybis[4,1-phenylene(1,3-dihydro-1,3-dioxo-2H-isoindole-2,5-diyl)carbonylimino]]bis- |
| 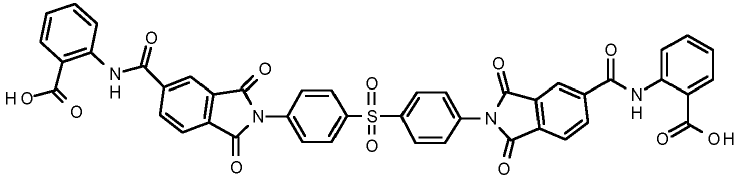 |
| 2-[[2-[4-[4-[5-[(2-carboxyphenyl)carbamoyl]-1,3-dioxoisoindol-2-yl]phenyl]sulfonylphenyl]-1,3-dioxoisoindole-5-carbonyl]amino]benzoic acid |
| 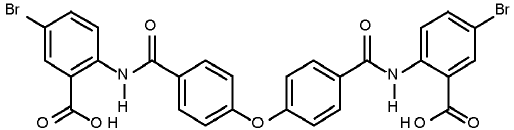 |
| 5-bromo-2-[[4-[4-[(4-bromo-2-carboxyphenyl)carbamoyl]phenoxy]benzoyl]amino]benzoic acid |
| 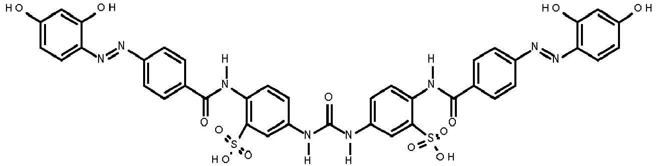 |
| Benzenesulfonic acid, 3,3'-(carbonyldiimino)bis[6-[[4-[(2,4-dihydroxyphenyl)azo]benzoyl]amino]- |
| 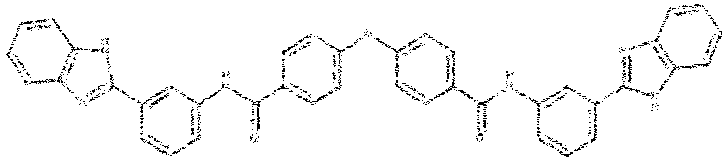 |
| N-[3-(1H-benzimidazol-2-yl)phenyl]-4-[4-[[3-(1H-benzimidazol-2-yl)phenyl]carbamoyl]phenoxy] benzamide |
| 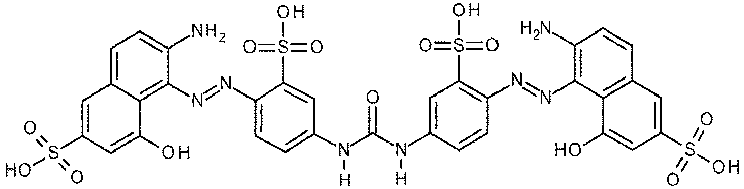 |
| 2-Naphthalenesulfonic acid, 5,5'-[carbonylbis[imino(2-sulfo-4,1-phenylene)-2,1-diazenediyl]]bis[6-amino-4-hydroxy- |
| 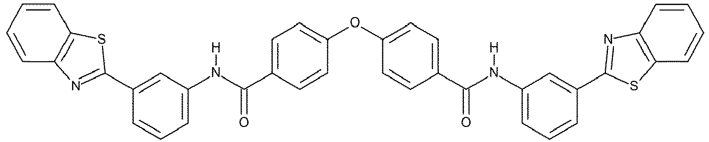 |
| N-[3-(1,3-benzothiazol-2-yl)phenyl]-4-[4-[[3-(1,3-benzothiazol-2-yl)phenyl]carbamoyl]phenoxy] benzamide |
| 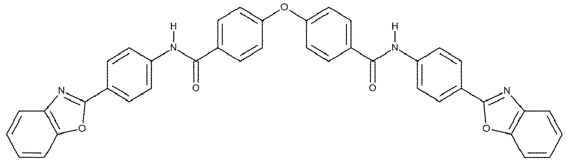 |
| Benzamide, 4,4'-oxybis[N-[4-(2-benzoxazolyl)phenyl]- |
| 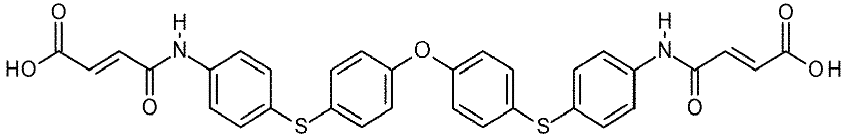 |
| (E)-4-[4-[4-[4-[4-[[(E)-3-carboxyprop-2-enoyl]amino]phenyl]sulfanylphenoxy]phenyl]sulfanylanilino]-4-oxobut-2-enoic acid |
| 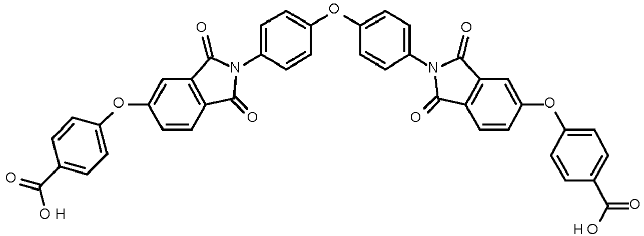 |
| 4-[2-[4-[4-[5-(4-carboxyphenoxy)-1,3-dioxoisoindol-2-yl]phenoxy]phenyl]-1,3-dioxoisoindol-5-yl] oxybenzoic acid |
| 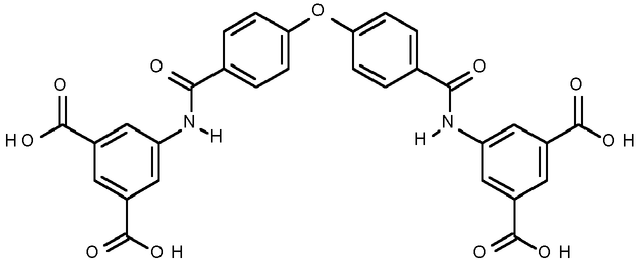 |
| 5-[[4-[4-[(3,5-dicarboxyphenyl)carbamoyl]phenoxy]benzoyl]amino]benzene-1,3-dicarboxylic acid |
| 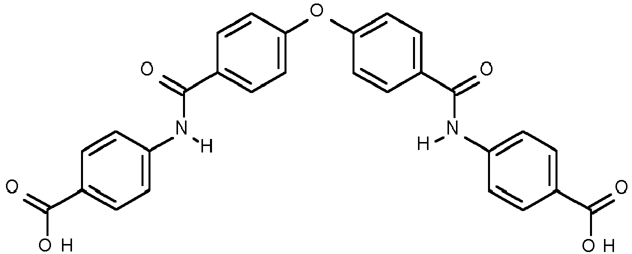 |
| 4,4'-[oxybis(benzene-4,1-diylcarbonylimino)]dibenzoic acid |
| 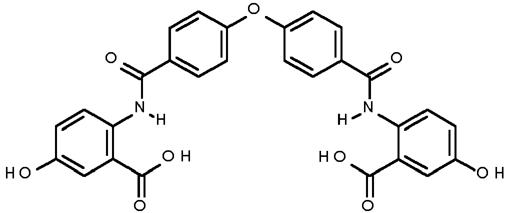 |
| Benzoic acid, 2,2'-[oxybis(4,1-phenylenecarbonylimino)]bis[5-hydroxy- |
| 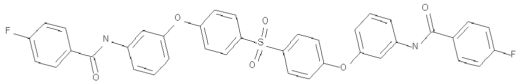 |
| Benzamide, N,N'-[sulfonylbis(4,1-phenyleneoxy-3,1-phenylene)]bis[4-fluoro- |
| 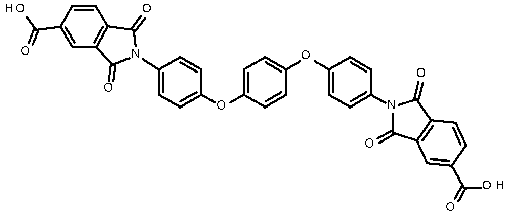 |
| 1H-Isoindole-5-carboxylic acid, 2,2'-[1,4-phenylenebis(oxy-4,1-phenylene)]bis[2,3-dihydro-1,3-dioxo- |
| 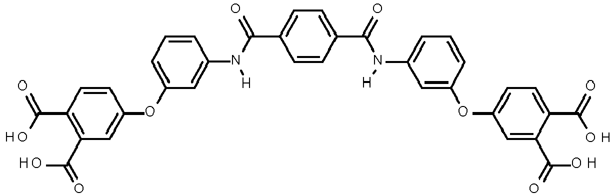 |
| 4-[3-[[4-[[3-(3,4-dicarboxyphenoxy)phenyl]carbamoyl]benzoyl]amino]phenoxy]phthalic acid |
| 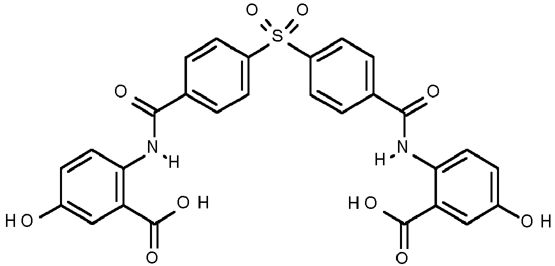 |
| Benzoic acid, 2,2'-[sulfonylbis(4,1-phenylenecarbonylimino)]bis[5-hydroxy |
| 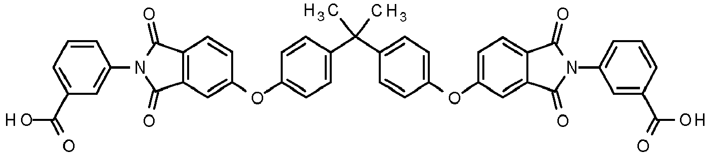 |
| 3-[5-[4-[2-[4-[2-(3-carboxyphenyl)-1,3-dioxoisoindol-5-yl]oxyphenyl]propan-2-yl]phenoxy]-1,3-dioxoisoindol-2-yl]benzoic acid |
| 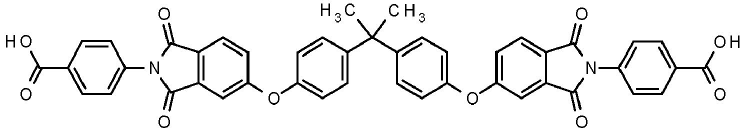 |
| 4-[5-[4-[2-[4-[2-(4-carboxyphenyl)-1,3-dioxoisoindol-5-yl]oxyphenyl]propan-2-yl]phenoxy]-1,3-dioxoisoindol-2-yl]benzoic acid |
| 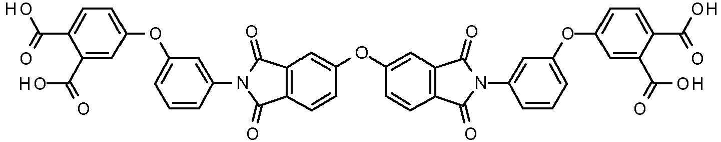 |
| 4-[3-[5-[2-[3-(3,4-dicarboxyphenoxy)phenyl]-1,3-dioxoisoindol-5-yl]oxy-1,3-dioxoisoindol-2-yl]phenoxy]phthalic acid |
| 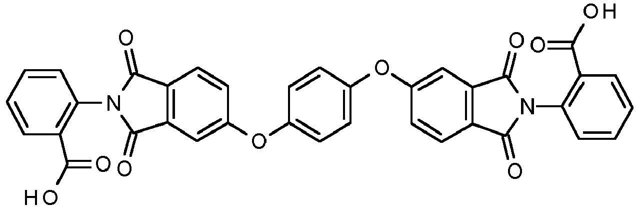 |
| Benzoic acid, 2,2'-[1,4-phenylenebis[oxy(1,3-dihydro-1,3-dioxo-2H-isoindole-5,2-diyl)]]bis- |
| 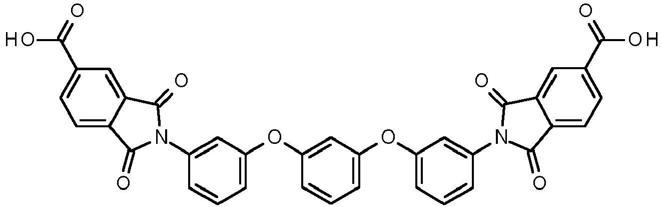 |
| 1H-Isoindole-5-carboxylic acid, 2,2'-[1,3-phenylenebis(oxy-3,1-phenylene)]bis[2,3-dihydro-1,3-dioxo- |
| 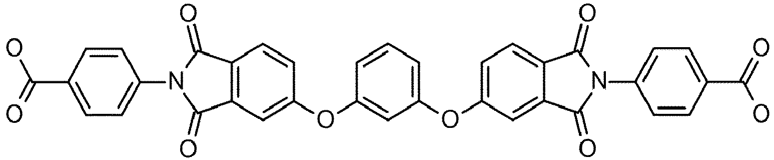 |
| Benzoic acid, 4,4'-[1,3-phenylenebis[oxy(1,3-dihydro-1,3-dioxo-2H-isoindole-5,2-diyl)]]bis- |
| 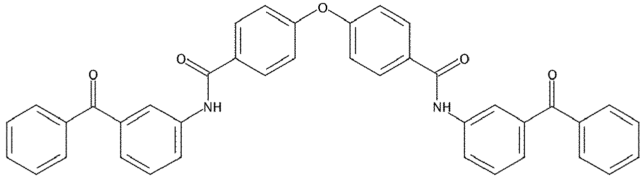 |
| Benzamide, 4,4'-oxybis[N-(3-benzoylphenyl)- |
| 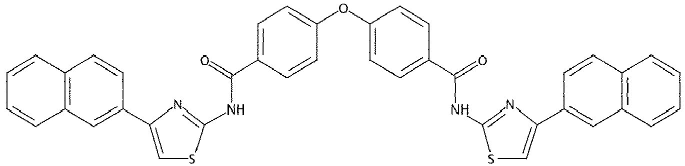 |
| Benzamide, 4,4'-oxybis[N-[4-(2-naphthalenyl)-2-thiazolyl]- |
| 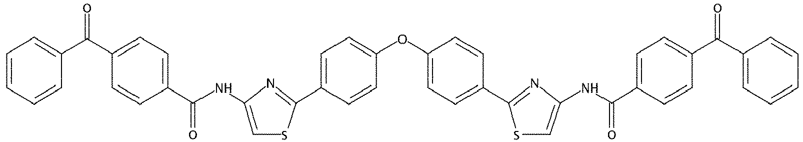 |
| Benzamide, N,N'-[oxybis(4,1-phenylene-2,4-thiazolediyl)]bis[4-benzoyl- |
| 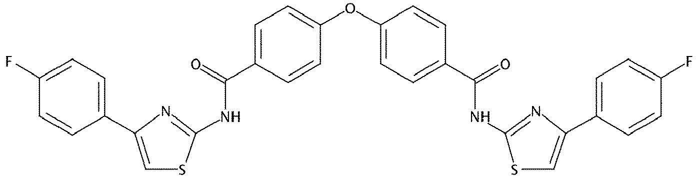 |
| Benzamide, 4,4'-oxybis[N-[4-(4-fluorophenyl)-2-thiazolyl]- |
| 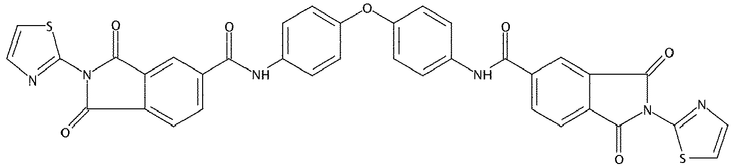 |
| 1H-Isoindole-5-carboxamide, N,N'-(oxydi-4,1-phenylene)bis[2,3-dihydro-1,3-dioxo-2-(2-thiazolyl)- |

11. The compound for the use according to claim 10 which is one of the followings:

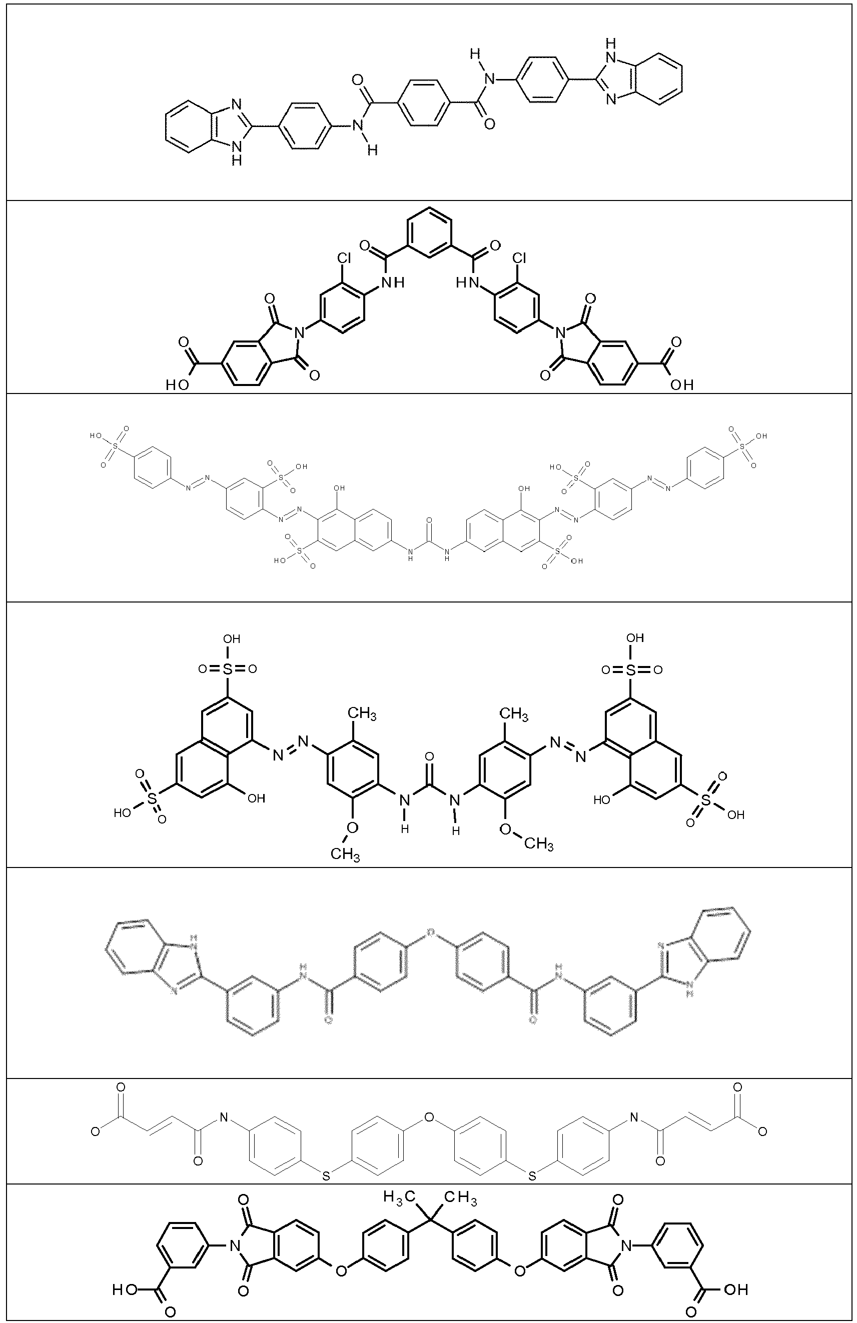

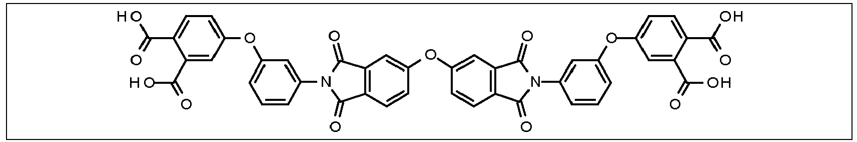

12. The compound for the use according to anyone of the preceding claims wherein said disease is selected from the group consisting of tumors, primary tumours, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis and aging, with the proviso that when said compound of formula (I) is 2-Naphthalenesulfonic acid, 7,7'-(carbonyldiimino)bis[4-hydroxy-3-[2-[2-sulfo-4-[2-(4-sulfophenyl)diazenyl]phenyl]diazenyl] or 2-Naphthalenesulfonic acid, 5,5'-[carbonylbis[imino(2-sulfo-4,1-phenylene)-2,1-diazenediyl]]bis[6-amino-4-hydroxy- said disease is not tumor or metastasis.

13. The compound of formula (I) according to anyone of claims 1-11 for use as an adjuvant of a chemotherapeutic agent.

14. A pharmaceutical composition containing as active ingredient a compound according to anyone of claims 1-11 and at least one pharmaceutically acceptable vehicle and/or excipient for use in the treatment of a disease which can be improved or prevented by an anti-heparanase activity.

15. The pharmaceutical composition according to claim 14 wherein said composition further comprises one or more further active ingredients.

16. The pharmaceutical composition according to claim 15 wherein said further active ingredient is a chemotherapeutic agent.

17. The pharmaceutical composition according to claim 16 wherein said chemotherapeutic agent is selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens.

18. The pharmaceutical composition according to claim 17 wherein said chemotherapeutic agent is a cytotoxic agent selected from the group consisting of melphalan, irinotecan, rituximab and lapatinib; or is a proteasome inhibitor selected from the group consisting of bortezomib, carfilzomib and ixazomib; or is an immunomodulatory agent selected from the group consisting of thalidomide, lenalidomide and pomalidomide.
